# EUROPEAN PATENT APPLICATION

(11) **EP 1 707 179 A1**
(43) Date of publication of application: **04.10.2006**
(21) Application number: 06005765.0
(22) Date of filing: 21.03.2006
(51) Int. Cl.: A61K 6/10

(54) **Impression material composition for recording occlusion**

(30) Priority: 31.03.2005 JP 2005103125
(71) Applicant: GC Corporation, Tokyo 174-8585 (JP)
(72) Inventor: Kamohara, Hiroshi, Itabashi-ku Tokyo 174-8585 (JP)
(74) Representative: Müller-Boré & Partner Patentanwälte

(57) **Abstract**

To provide an impression material composition for recording occlusion, which has high strength while keeping characteristics of the conventional impression material for recording the occlusion, in which setting characteristics is excellent, hardness of a set material is high, and the machinability is high, the impression material composition for recording occlusion comprises (A) 100 weight parts of organopolysiloxane having at least two aliphatic unsaturated groups in one molecule and having viscosity of 50 to 600 mPa·s at 25°C; (B) 0.1-30 weight parts of organohydrogenpolysiloxane having at least two HSiR₂O_{1/2} units in one molecule and/or organohydrogenpolysiloxane having an HSiR₂O_{1/2} unit and anSiO_{1/2}unit; (C) 0.1-20 weight parts of silicone resin having a specific molecular weight in which the repeating units comprise Rl₃SiO_{1/2} and SiO_{1/2}, and a mol ratio of Rl₃SiO_{1/2} unit with respect to SiO_{1/2} unit (Rl₃SiO_{1/2}/ SiO_{1/2}) is 0.4 to 2.0, (D) a silicone-soluble platinum compound in an amount of 10-500ppm; and (E) 20-500 weight parts of inorganic filler.

## Description

The present invention relates to an impression material composition for recording an occlusion, and more particularly, relates to the impression composition for recording the occlusion having high speed setting characteristics obtaining, high hardness of a set body and high strength, and being useful in both of physical property and operativity.

When a prosthesis such as a crown, a bridge or the like is produced, it is necessary to accurately record an occlusion relation of a patient, and accurately and rapidly reproduce the occlusion relation of the model produced by recording the occlusion on a dental articulator. As a material used for recording the occlusion relation, there is a wax, a gypsum, a zinc oxide-eugenol-based impression material, a thermoplastic synthetic resin compound, a polyether impression material, a silicone impression material or the like.

In these materials, since the wax is interposed between occlusal surfaces to thereby collect the occlusion, it is hard to guide a jaw position of a patient to a centric occlusion position. Further, the wax is easily deformed at the time of mounting on the dental articulator. Further, when the wax after recording the occlusion is sent to a dental laboratory, it may be deformed because of its low strength. As for the gypsum and the zinc oxide-eugenol-based impression material, these are weak in quality of the material, so that they must be handle with care, and their operativities are inferior. Further, since machinabilities are insufficient, it is hard to mount those on the dental articulator. As for the thermoplastic synthetic resin compound, it is necessary to operate it by heating, so that the operation is complicated. Further, the machinability is also insufficient. On the other hand, as for the polyether impression material and the silicone impression material, handling is easy, but there are problems that setting characteristics is insufficient, and these are deformed easily according to time when removing the set material body from the inside of an oral cavity of a patient. Then, a silicone impression material only for recording the occlusion has been developed (for example, refer to Japanese Patent Application Laid Open No. 7(1995)-112911). However, there is a problem that the hardness of the set material is insufficient, and it is easily broken.

An obj ective of the present invention is to provide the impression material composition for recording the occlusion, which has high strength while keeping characteristics of the conventional impression material for recording the occlusion, in which setting characteristics is excellent, hardness of a set material is high, and machinability is high.

The earnest work was carried out in order to solve the above-mentioned problems and, as a result of this, it was found out that, when a silicone resin comprising repeating units of an M unit (Rl₃SiO_{1/2}) and a Q unit (SiO_{1/2}), where the M unit and the Q unit are contained in specific ratio, is used together with organopolysiloxane having specific viscosity and specific organohydrogenpolysiloxane, the strength can be enhanced without damaging the excellent abilities such as the setting characteristics or the like of the conventional impression material for recording the occlusion.

That is, the impression material composition for recording the occlusion according to the present invention comprises (A) 100 weight parts of organopolysiloxane having at least two aliphatic unsaturated groups in one molecule and having viscosity of 50 to 600 mPa·s at 25°C; (B) 0.1-30 weight parts of organohydrogenpolysiloxane having at least two HSiR₂O_{1/2} units in one molecule and/or organohydrogenpolysiloxane having an HSiR₂O_{1/2} unit and an SiO_{1/2} unit (R is a substituted or unsubstituted hydrocarbon group having 1-10 carbon atoms) ; (C) 0.1-20 weight parts of silicone resin in which the repeating units comprise an M unit being Rl₃SiO_{1/2} (Rl is a substituted or unsubstituted, same kinds or different kinds of monovalent hydrocarbon group) and a Q unit being SiO_{1/2}, a mol ratio of the M unit with respect to the Q unit (M/Q) is 0.4 to 2.0 and molecular weight is 500 to 100,000; (D) a silicone-soluble platinum compound in an amount of 10-500ppm (based on the total amount of the components (A) to (C)); and (E) 20-500 weight parts of inorganic filler.

The present invention is the impression material composition for recording the occlusion having the excellent property against breakage, with having additionally the characteristics of the conventional material for which setting characteristic is excellent, hardness of a set material is high and machining is easy.

The organopolysiloxane which is the component(A) used for the impression material composition for recording the occlusion according to the present invention is a low viscosity organopolysiloxane having at least two aliphatic unsaturated groups in one molecule and having viscosity of 50 to 600 mPa·s at 25°C. The viscosity of the organopolysiloxane is 50 to 600 mPa·s. If the viscosity is less than 50 mPa· s, it is easily evaporated at room temperature, and if the viscosity is more than 600 mPa·s, the hardness of the cured body is decreased, the machinability is also decreased, and further, viscosity of a paste is increased, to thereby increase resistance at the time of recording the occlusion.

It is preferable that the organopolysiloxane is linear chain type and both molecular chain terminals are blocked with a vinylsilyl group. The number of the vinyl group at the terminal may be two or more, and the vinyl group may be contained in the molecular chain. This organopolysiloxane is commonly called as a silicone oil and it is already known that the silicone oil has a fixed molecular weight distribution in general. However, in the present invention, the molecular weight distribution is not necessarily be limited especially, and a mixture of one or more kinds of the organopolysiloxane containing the aliphatic unsaturated groups may be used if the viscosity thereof at 25°C is 50 to 600 mPa·s .

When the second component (B) of the organohydrogenpolysiloxane having at least two HSiR₂O_{1/2} units in one molecule and/or the organohydrogenpolysiloxane having the HSiR₂O_{1/2} unit and the SiO_{1/2} unit is used as a crosslink material, this can give the characteristics of the impression material for recording the occlusion in which a short holding time in the oral cavity and high speed setting are needed, and this property is different from that of the general dental impression material. More particularly, when these materials are combined with the component (C) described below, the strength can be enhanced without changing the setting characteristics. In the above chemical symbol, R is the substituted or unsubstituted hydrocarbon group having 1-10 carbon atoms. When the organohydrogenpolysiloxane containing the HSiR₂O_{1/2} unit and the SiO_{1/2} unit is used, the setting speed is increased, and the hardness is also increased, so that it is preferably used for the impression material for recording the occlusion. As for the organohydrogenpolysiloxane, if the weight parts thereof are less than 0.1 with respect to the 100 weight part of component (A), the hardness of the set material is decreased, and the setting speed is also decreased. Further, if the weight parts are more than 30, the setting speed is high, but the set material is remarkably weak.

As for the third component (C), the silicone resin in which the repeating units comprising the M unit being Rl₃SiO_{1/2} (Rl is substituted or unsubstituted, same kinds or different kinds of monovalent hydrocarbon group) and the Q unit being SiO_{1/2}, the mol ratio of the M unit to the Q unit (M/Q) is 0.4 to 2.0 and molecular weight is 500 to 100,000, has excellent compatibility and dispersibility with an organic resin such as the other organopolysiloxane combined in the impression material composition for recording the occlusion according to the present invention. Thus, when this resin is used together with the components (A) and (B) of the present invention, the strength can be enhanced while keeping the same characteristics as those of the conventional impression material for recording the occlusion. The silicone resin which is the component (C) may contain 1 to 20 mol % of a T unit being R2SiO_{3/2} (where R2 is substituted or unsubstituted, same kinds or different kinds of monovalent hydrocarbon group, and 30 mol % or more of this group have two or more carbon atoms.) In the case of containing the T unit, if the mol % is less than 1%, there is not remarkable effect in the ability of the impression material composition for recording the occlusion, and if it is more than 20% the silicone resin may become unstable and be adhered or gelled in a production process.

The silicone resin, which is the (C) component, comprises the M, T and Q units. As for a polymerizing ratio of this resin, the mol ratio of the M unit with respect to the Q unit (M/Q) is 0.4 to 2.0. If the M/Q is less than 0.4, the compatibility and the dispersibility with respect to the organic resin such as the organopolysiloxane are insufficient, where the organopolysiloxane is combined in the impression material composition for recording the occlusion according to the present invention. Further, if it is more than 2.0, the strength and the other physical property of the composition are not enhanced. Further, if the molecular weight is less than 500, the effect cannot be obtained by blending the resin, and if it is more than 100, 000, the viscosity of the composition is remarkably high, so that the operation becomes hard.

As for the blending amount of the silicone resin, in which the repeating units used in the present invention comprise the M unit and the Q unit, the mol ratio of the M unit to the Q unit (M/Q) is 0.4 to 2.0, and the molecular weight is 500 to 100,000, the weight parts thereof are 0.1 to 20 with respect to 100 weight parts of component (A), and preferably 0.5 to 5. If the weight parts are less than 0.1, the sufficient ability cannot be obtained, and if the weight parts are more than 20, the setting characteristics is worsen, and the high ability cannot be obtained.

As the silicone-soluble platinum compound which is the fourth component (D), chloroplatinic acid, alcohol-modified chloroplatinic acid, a complex of chloroplatinic acid and olefin or the like, which are a publicly-known addition reaction catalyst, can be used. Especially, a vinylsiloxane complex of chloroplatinic acid is preferably used. This is obtained by substituting chlorine in the chloroplatinic acid to vinylsiloxane. More particularly, a compound in which 1,3 divinyltetramethyldisiloxane or vinylmethylpolysiloxane containing a vinyl group at a side chain is made into a ligand of a platinum atom can be used (as disclosed in Japanese Patent Publication No.48-10947). The additional amount of this compound is within the range of 10 to 500 ppm with respect to the total amount of the components (A) to (C). If the addition amount is less than 10 ppm, there are problems that the setting speed is insufficient, and the setting speed is more decreased when a little amount of a substance, which prevents catalytic ability of the platinum compound, exists. Further, if the additional amount is more than 500 ppm, the curing speed is increased too much, and it is necessary to use much amount of a controlling agent for adjusting the setting speed, so that the quality becomes instable. Further, since much amount of the platinum catalyst having high cost is used, it is economically disadvantageous. It is preferable that the silicone-soluble platinum compound is used by dissolving with an alcohol based, ketone based, ether based, or hydrocarbon based solvents, a silicone oil or the like.

As the inorganic filler which is used as the fifth component (E), quartz, cristobalite, diatomaceous earth, fused quartz, glass fiber, titanium dioxide, fumed silica or the like can be used. The inorganic filler may be used 20 to 500 weight parts with respect to the 100 weight parts of component (A) . If the weight parts are less than 20, the set material becomes weak, and if the weight parts are more than 500, the viscosity of the composition is increased too much to thereby increase the resistance at the time of kneading and recording the occlusion. Thus, the high accuracy occlusion record cannot be carried out. Furthermore, various kinds of organic or inorganic coloring agents may be used, within the range that the characteristics of the impression material composition for recording the occlusion according to the present invention are not lost. As these coloring agents, a coloring agent used for a general silicone composition such as red iron oxide, titanium white, titanium yellow, cobalt blue or the like is used.

Then, the present invention will be explained concretely with examples, but the present invention is not limited to these examples. In the examples, parts shows weight parts, and viscosity shows a measured value at 25°C .

### [Example]

### <Example 1>

A base paste and a catalyst paste having the following compositions were produced.

### (Base Paste)

A) 100 weight parts of dimethylpolysiloxane in which the viscosity was 70 mPa·s, and both molecular chain terminals were blocked with a dimethylvinylsiloxy group.
B) 5 weight parts of branched methylhydrogenpolysiloxane in which both molecular chain terminals were blocked with a dimethylhydrogensilyl group.
C) 1 weight parts of silicone resin in which the repeating units comprised the M unit and the Q unit, the mol ratio of the M unit with respect to the Q unit (M/Q) was 1.0, 8 mol % of the T unit was contained, and the molecular weight was 10,000.
E) 200 weight parts of quartz powder.

### (Catalyst Paste)

A) 100 weight parts of dimethylpolysiloxane in which the viscosity was 70 mPa·s, and both molecular chain terminals were blocked with a dimethylvinylsiloxy group.
B) 5 weight parts of branched methylhydrogenpolysiloxane in which the both molecular chain terminals were blocked with the dimethylhydrogensilyl group.
D) 2 weight parts of silicone oil solution containing 0.2 weight % of platinum complex.
E) 200 weight parts of quartz powder.

Then, equivalent amounts of the base paste and the catalyst paste were mixed, and kneaded by a spatula for 20 seconds and thereafter, the following tests were carry out.

### <Evaluation of hardness of the set material>

The hardness of the set material after 8 minutes from start of the kneading was measured by a method according the JIS K-6253Atype. These results were shown in Table 1.

### <Machinability of the set material>

The machinability of the set material was measured by cutting the set material with a knife corresponding to a clinical operation, and sensuously comparing the easiness of cutting. These results were shown in Table 1.

### <Evaluation of ability against breakage>

A test piece having a width of 1mm, a length of 80 mm and a thickness of 1 mm was cut out from the set material, and the test piece was softly deformed by hand at the middle of the length so as to have a U shape, to thereby confirm occurrence of the breakage of the test piece or not. These results were shown in Table 1.

### <Example 2>

The base paste and the catalyst paste having the following compositions were produced.

### (Base Paste)

A) 100 weight parts of dimethylpolysiloxane in which the viscositywas 500 mPa·s, and the bothmolecular chain terminals were blocked with the dimethylvinylsiloxy group.
B) 10 weight parts of branched methylhydrogenpolysiloxane inwhich the bothmolecular chain terminals were blocked with the dimethylhydrogensilyl group.
C) 1 weight parts of silicone resin in which the repeating units comprised the M unit, the Q unit and the T unit, the mol ratio of the M unit with respect to the Q unit (M/Q) was 1.5, 10 mol % of T unit was contained, and the molecular weight is 15,000.
E) 500 weight parts of fused quartz powder.

### (Catalyst Paste)

A) 100 weight parts of dimethylpolysiloxane in which the viscosity was 500 mPa·s, and the both molecular chain terminals were blocked with the dimethylvinylsiloxy group.
C) 1 weight parts of silicone resin in which the repeating units comprised the M unit, the Q unit and the T unit, the mol ratio of the M unit with respect to the Q unit (M/Q) was 1.0 , 8 mol % of T unit was contained, and the molecular weight is 10,000.
D) 10 weight parts of octyl alcohol solution containing 0.5 weight % of chloroplatinic acid.
E) 500 weight parts of fused quartz powder.

Then, equivalent amounts of the base paste and the catalyst paste were mixed, and kneaded with a spatula for 20 seconds and thereafter, the same tests as those of Example 1 were carried out.

### <Example 3>

The base paste and the catalyst paste having the following compositions were produced.

### (Base Paste)

A) 100 weight parts of dimethylpolysiloxane in which the viscosity was 100 mPa·s, and the both molecular chain terminals were blocked with the dimethylvinylsiloxy group.
B) 5 weight parts of methylhydrogenpolysiloxane containing the HSiR₂O_{1/2} unit and the SiO_{1/2} unit.
C) 1 weight parts of silicone resin in which the repeating units comprised the M unit, the Q unit and the T unit, the mol ratio of the M unit with respect to the Q unit (M/Q) was 0.5, 11 mol % of T unit was contained, and the molecular weight is 30,000.

### (Catalyst Paste)

A) 100 weight parts of dimethylpolysiloxane in which the viscosity was 100 mPa·s, and the both molecular chain terminals were blocked with the dimethylvinylsiloxy group.
D) 3 weight parts of silicone oil solution containing 0.4 weight % of 1,3 divinyltetramethyldisiloxane and platinum complex.
E) 100 weight parts of quartz powder.

Then, equivalent amounts of the base paste and the catalyst paste were mixed, and kneaded with a spatula for 20 seconds and thereafter, the same tests as those of Example 1 were carried out.

### <Example 4>

The base paste and the catalyst paste having the following compositions were produced.

### (Base Paste)

A) 100 weight parts of dimethylpolysiloxane in which the viscosity was 150 mPa·s, and the both molecular chain terminals were blocked with the dimethylvinylsiloxy group.
B) 20 weight parts of methylhydrogenpolysiloxane containing the HSiR₂O_{1/2} unit and the SiO_{1/2} unit.
C) 3 weight parts of silicone resin in which the repeating units comprised the M unit, the Q unit and the T unit, the mol ratio of the M unit with respect to the Q unit (M/Q) was 3.1, 0.5 mol % of T unit was contained, and the molecular weight is 5,000.
E) 100 weight parts of quartz powder.

### (Catalyst Paste)

A) 100 weight parts of dimethylpolysiloxane in which the viscositywas 100 mPa·s, and the both molecular chain terminals were blocked with the dimethylvinylsiloxy group.
D) 3 weight parts of silicone oil solution containing 0.4 weight % of 1,3 divinyltetramethyldisiloxane and platinum complex.
E) 100 weight parts of fused quartz powder.

Then, equivalent amounts of the base paste and the catalyst paste were mixed, and kneaded with a spatula for 20 seconds and thereafter, the same tests as those of Example 1 were carried out.

### <Example 5>

The base paste and the catalyst paste having the following compositions were produced.

### (Base Paste)

A) 100 weight parts of dimethylpolysiloxane in which the viscosity was 500 mPa·s, and the both molecular chain terminals were blocked with the dimethylvinylsiloxy group.
B) 10 weight parts of branched methylhydrogenpolysiloxane in which the both molecular chain terminals were blocked with the dimethylhydrogensilyl group.
E) 500 weight parts of fused quartz powder.

### (Catalyst Paste)

A) 100 weight parts of dimethylpolysiloxane in which the viscosity was 500 mPa·s, and the both molecular chain terminals were blocked with the dimethylvinylsiloxy group.
C) 1 weight parts of silicone resin in which the repeating units comprised the M unit, the Q unit and the T unit, the mol ratio of the M unit with respect to the Q unit (M/Q) was 1.0, 8 mol % of T unit was contained, and the molecular weight is 10,000.
C) 1 weight parts of silicone resin in which the repeating units comprised the M unit, the Q unit and the T unit, the mol ratio of the M unit with respect to the Q unit (M/Q) was 1.5, 10 mol % of T unit was contained, and the molecular weight is 15,000.
D) 10 weight parts of octyl alcohol solution containing 0.5 weight % of chloroplatinic acid.
E) 500 weight parts of fused quartz powder.

Then, equivalent amounts of the base paste and the catalyst paste were mixed, and kneaded with a spatula for 20 seconds and thereafter, the same tests as those of Example 1 were carried out.

### <Comparison example 1>

The same base paste and the catalyst paste as those of Example 1, where the C) component is excepted, were used for Comparison example 1. These pastes were kneaded like Example 1 and thereafter, the same tests as those of Example 1 were carried out.

### <Comparison example 2>

In Comparison example 2, the viscosity of the component A) is different.

### (Base Paste)

A) 100 weight parts of dimethylpolysiloxane in which the viscosity was 3000 mPa·s, and the both molecular chain terminals were blocked with the dimethylvinylsiloxy group.
B) 5 weight parts of linear chain methylhydrogenpolysiloxane containing 40 mol % of the methylhydrogenpolysiloxane unit.
C) 1 weight parts of silicone resin in which the repeating units comprised the M unit, the Q unit and the T unit, the mol ratio of the M unit with respect to the Q unit (M/Q) was 1.0, 8 mol % of T unit was contained, and the molecular weight is 10,000.
E) 300 weight parts of quartz powder.

### (Catalyst Paste)

A) 100 weight parts of dimethylpolysiloxane in which the viscosity was 33000 mPa·s, and the both molecular chain terminals were blocked with the dimethylvinylsiloxy group.
D) 10 weight parts of octyl alcohol solution containing 0.5 weight % of chloroplatinic acid.
E) 200 weight parts of quartz powder.

These pastes were kneaded like Example 1 and thereafter, the same tests as those of Example 1 were carried out.

### <Comparison example 3>

In Comparison example 3, the component E) is not contained.

### (Base Paste)

A) 100 weight parts of dimethylpolysiloxane in which the viscosity was 70 mPa·s, and the both molecular chain terminals were blocked with the dimethylvinylsiloxy group.
B) 5 weight parts of branched methylhydrogenpolysiloxane in which the both molecular chain terminals were blocked with the dimethylhydrogensilyl group.
C) 1 weight parts of silicone resin in which the repeating units comprised the M unit, the Q unit and the T unit, the mol ratio of the M unit with respect to the Q unit (M/Q) was 1.0, 8 mol % of T unit was contained, and the molecular weight is 10,000.

### (Catalyst Paste)

A) 100 weight parts of dimethylpolysiloxane in which the viscosity was 70 mPa·s, and the both molecular chain terminals were blocked with the dimethylvinylsiloxy group.
B) 5 weight parts of branched methylhydrogenpolysiloxane in which the both molecular chain terminals were blocked with the dimethylhydrogensilyl group.
D) 2 weight parts of silicone oil solution containing 0.2 weight % of platinum complex.

**Table 1**

| | Example 1 | Example 2 | Example 3 | Example 4 | Example 5 | Comparison example 1 | Comparison example 2 | Comparison example 3 |
|---|---|---|---|---|---|---|---|---|
| Hardness | 92 | 90 | 95 | 92 | 94 | 92 | 90 | 65 |
| Machinability | good | good | good | good | good | good | Not good | Not good |
| Strength against breakage | No breakage | No breakage | No breakage | No breakage | No breakage | Breakage | Breakage | No breakage |

As clearly known from the Table 1, the hardness and the machinability are equal but the strength is enhanced in the impression material composition for recording the occlusion according to the present invention, as compared with Comparison example 1 in which the conventional material for is used. Further, as for Comparison example 2, the strength is low, and the viscosity of the composition before hardening is too high, so that it cannot be used as the impression material for recording the occlusion.

## Claims

1. An impression material composition for recording occlusion, comprising,
(A) 100 weight parts of organopolysiloxane having at least two aliphatic unsaturated groups in one molecule and having viscosity of 50 to 600 mPa·s at 25°C;
(B) 0.1-30 weight parts of organohydrogenpolysiloxane having at least two HSiR₂O_{1/2} units in one molecule and/or organohydrogenpolysiloxane having an HSiR₂O_{1/2} unit and an SiO_{1/2} unit, where R is a substituted or unsubstituted hydrocarbon group having 1-10 carbon atoms;
(C) 0.1-20 weight parts of silicone resin in which the repeating units comprise an M unit being Rl₃SiO_{1/2}, where Rl is substituted or unsubstituted same kinds or different kinds of monovalent hydrocarbon group, and a Q unit being SiO_{1/2}, a mol ratio of the M unit with respect to the Q unit (M/Q) is 0.4 to 2.0, and molecular weight is 500 to 100,000;
(D) a silicone-soluble platinum compound in an amount of 10-500ppm based on the total amount of the components (A) to (C); and
(E) 20-500 weight parts of inorganic filler.
